# EUROPÄISCHE PATENTSCHRIFT

(11) **EP 0 589 335 B1**
(45) Veröffentlichungstag und Bekanntmachung des Hinweises auf die Patenterteilung: **18.06.2003**
(21) Anmeldenummer: 93114772.2
(22) Anmeldetag: 14.09.1993
(51) Int. Cl.: C07D 487/04, A61K 31/505

(54) **N1-Substituierte 1H-1,2,3-Triazolo[4,5-d]pyrimidine, Verfahren zu ihrer Herstellung sowie ihre Verwendung als antivirale Mittel**
N1-Substituted- (1H)-1,2,3-Triazolo (4,5-d) pyrimidines, process for their preparation and their use as antiviral means
(1H)-1,2,3-Triazolo(4,5-d) pyrimidines substituées en N1, procédé pour leur préparation de même que leur utilisation comme moyens antiviraux

(30) Priorität: 24.09.1992 DE 4231944
(43) Veröffentlichungstag der Anmeldung: 30.03.1994
(73) Patentinhaber: HOECHST AKTIENGESELLSCHAFT, 65929 Frankfurt am Main (DE)
(72) Erfinder: Jähne, Gerhard, Dr., D-65929 Frankfurt am Main (DE); Helsberg, Matthias, Dr., D-65779 Kelkheim/Ts. (DE); Winkler, Irvin, Dr., D-65835 Liederbach (DE); Gross, Gerhard, Dr., D-65439 Flörsheim am Main (DE); Scholl, Thomas, Dr., D-53113 Bonn (DE)

(56) Entgegenhaltungen:
- EP-A- 0 201 289
- EP-A- 0 452 680
- EP-A- 0 464 511
- WO-A-90/06312
- US-A- 4 268 672
- US-A- 4 543 255
- PATENT ABSTRACTS OF JAPAN vol. 8, no. 187 (C-240)28. August 1984
- J. MED. CHEM. Bd. 28, Nr. 8, 1985, WASHINGTON US Seiten 982 - 987 L.M. BEAUCHAMP ET AL.
- BIOCHEMICAL PHARMACOLOGY Bd. 36, Nr. 8, 1987, Seiten 1237 - 1244 J.M. STEIN ET AL.
- CHEMICAL ABSTRACTS, vol. 75, no. 13, 27. September 1971, Columbus, Ohio, US; abstract no. 88567x, A. COLAUTTI ET AL.
- CHEMICAL ABSTRACTS, vol. 118, no. 7, 15. Februar 1993, Columbus, Ohio, US; abstract no. 60018b, F. SEELA ET AL.

## Beschreibung

Die vorliegende Erfindung betrifft Derivate des 1H-1,2,3-Triazolo[4,5-d]pyrimidins, die in 1-Stellung einen Alkoxymethylrest tragen, Verfahren zur Herstellung dieser Verbindungen sowie ihre Verwendung als antivirale Mittel.

Des weiteren betrifft die Erfindung die physiologisch verträglichen Salze der genannten Verbindungen.

Während die antivirale Wirksamkeit und die Herstellung von Purinnukleosidanaloga, die in 9-Stellung einen acyclischen Rest tragen, schon lange bekannt ist (siehe z. B. DE-OS 2539963 oder K. K. Ogilvie et al., Can. J. Chem. 62, 241 (1984) oder C. K. Chu und S. J. Cutler, J. Heterocyclic Chem. 23, 289 (1986)) und auch die Herstellung einzelner Verbindungen mit einem acyclischen Rest in der 3-Position des 5-Amino-7-hydroxy-3H-1,2,3-triazolo[4,5-d]pyrimidins beschrieben ist (siehe L M. Beauchamp et al., J. Med. Chem. 28, 982 (1985) oder J. M. Stein et al., Biochem. Pharmacol. 36, 1237 (1987)), ist über die Synthese von
1H-1,2,3-Triazolo[4,5-d]pyrimidinen, die in 1-Stellung einen acyclischen Rest tragen, nur wenig bekannt (siehe Chemical Abstracts 53, 1389g (1959) und L M. Beauchamp et al., J. Med. Chem. 28, 982 (1985)) und über eine antivirale Wirkung solcher Verbindungen, bei denen der acyclische Rest einen eventuell substituierten 2-Hydroxyethoxymethyl- oder 1,3-Dihydroxy-2-propoxymethyl- oder 2,3-Dihydroxy-1-propoxymethyl-Rest darstellt, wurde bisher nicht berichtet.

Es wurde nun überraschenderweise gefunden, daß bestimmte 1-substituierte 1H-1,2,3-Triazolo[4,5-d]pyrimidine und deren physiologisch verträgliche Salze, antivirale Eigenschaften, insbesondere gegen verschiedene DNA-, RNA- und Retroviren aufweisen.

Erfindungsgegenstand ist demzufolge die Verbindung der Formel I 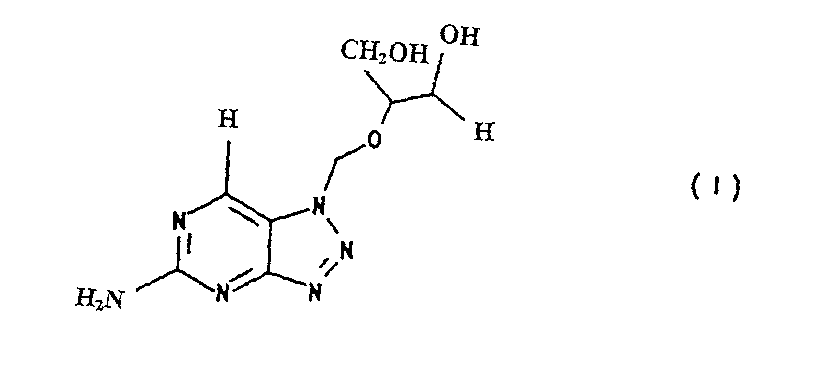 der tautomere Formen sowie deren physiologisch verträgliche Salze.

Besonders für therapeutische Zwecke geeignete Salze der erfindungsgemäßen Verbindungen sind Salze von physiologisch verträglichen organischen und anorganischen Säuren wie Essigsäure, Milchsäure, Äpfelsäure, p-Toluolsulfonsäure, Methansulfonsäure, Isäthionsäure, Salzsäure oder Schwefelsäure.

Zum Erfindungsgegenstand gehört weiterhin die Verwendung der genannten Verbindungen als Arzneimittel. Besonders wirksam sind die erfindungsgemäßen Verbindungen gegen Herpes Simplex Viren Typ 1 und Typ 2, Cytomegalie Viren, Varicella Zoster Viren, Epstein-Barr Viren und das Humane Herpes Virus Typ 6 (HHV 6).

Darüber hinaus gehört zum Gegenstand der vorliegenden Erfindung die Verwendung der obengenannten Verbindungen der Formel (I) zur Herstellung von Arzneimitteln zur Prophylaxe oder zur Behandlung von Viruserkrankungen.

Weiterhin betrifft die vorliegende Erfindung ein Verfahren zur regioselektiven Herstellung von in 1-Stellung substituierten 1H-1,2,3-Triazolo[4,5-d]pyrimidinen der Formel I oder eines physiologisch verträglichen Salzes derselben, dadurch gekennzeichnet, daß man eine Verbindung der Formel II, 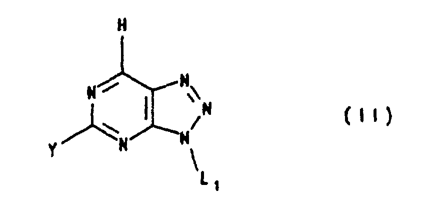 worin
- Y =: Trialkylsilylamino, bevorzugt Trimethylsilylacetamido,
und die austretende Gruppe L₁ = Acyloxy oder Trialkylsilyl, bevorzugt Trimethylsilyl, bedeuten
mit einer Verbindung der Formel III, 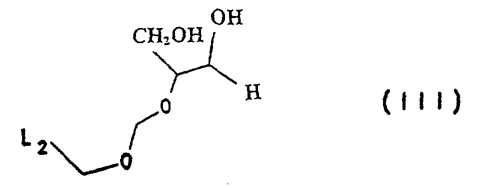 worin
die austretende Gruppe L₂ = Halogen, bevorzugt Chlor, oder die C₁-C₆-Alkylthio- oder C₁-C₆-Alkylsulfinyl- oder C₁-C₆-Alkylsulfonyl-Gruppierung, bevorzugt die Methylthiooder die Methylsulfinyl- oder die Methylsulfonyl-Gruppierung, oder C₁-C₈-Acyloxy oder Benzoyloxy, bevorzugt Acetoxy, oder aber
-O-CHR¹-CHR²R³ bedeuten (wobei in diesem Fall die Verbindung der Formel III ein symmetrische Formaldehydacetal darstellt),
in einem aprotischen Lösungsmittel wie Benzol, Toluol, Xylol, Acetonitril, Dichlormethan oder 1,2-Dichlorethan oder Gemischen davon,
unter einer Schutzgasatmosphäre aus Argon oder Stickstoff, in Gegenwart einer Säure, bevorzugt einer Lewis-Säure wie Aluminiumtrichlorid, Aluminiumsulfat, Bortrifluorid, Eisentrichlorid, Galliumtrichlorid, Zinntetrachlorid oder Titantetrachlorid oder in Gegenwart von Jod oder vorzugsweise Carbonsäure- oder Alkylsulfonsäuretrialkylsilylestern, besonders Trifluormethansulfonsäuretrimethylsilylester, wobei die Mengen dieser Reagenzien 0.01 bis 10, vorzugsweise 0.3 bis 1.3 Äquivalente, bezogen auf die Menge der jeweils eingesetzten Verbindung der Formel II, betragen,
bei Temperaturen zwischen -70°C und +40°C, vorzugsweise zwischen -40°C und +20°C,
während 1 bis 24 Stunden, vorzugsweise während 2-8 Stunden,
umsetzt.
Dieses Verfahren liefert in guter Regioselektivität, in der Regel >4 : 1, bevorzugt das 1H-1-Isomer des jeweiligen 1,2,3-Triazolo[4,5-d]pyrimidins.

Eine bevorzugte Verbindung der Formel II ist gegeben durch X = -OSi(CH₃)₃, Y = CH₃C(O)N(Si(CH₃)₃)- und L₁ = -Si(CH₃)₃.
Bevorzugte Verbindungen der Formel III sind gegeben durch L₂ = -O-CHR¹-CHR²R³,
wobei R¹ = Isopropoxymethyl oder Wasserstoff, R² = Isopropoxy oder Wasserstoff und R³ = Isopropoxymethyl oder Wasserstoff ist.

Die erfindungsgemäßen Verbindungen der Formel I können in der acyclischen Seitenkette ein oder mehrere chirale Zentren aufweisen. Die Verbindungen liegen in der Regel als Racemate vor; eine Herstellung bzw. Isolierung der reinen Enantiomere ist möglich. Gegenstand der Erfindung sind deshalb sowohl die reinen Enantiomeren als auch Mischungen derselben, wie z. B. das zugehörige Racemat.

Des weiteren betrifft die vorliegende Erfindung Arzneimittel mit einem Gehalt an mindestens einer erfindungsgemäßen Verbindung.

Die erfindungsgemäßen Arzneimittel können enteral (oral), parenteral (intravenös), rektal oder lokal (topisch) angewendet werden. Sie können in Form von Lösungen, Pulvern (Tabletten, Kapseln einschließlich Mikrokapseln), Salben (Cremes oder Gel) oder Suppositorien verabreicht werden. Als Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage. Als zweckmäßige Dosierung werden 0.1 - 10, vorzugsweise 0.2 - 8 mg/kg Körpergewicht verabreicht. Sie werden zweckmäßig in Dosierungseinheiten verabreicht, die mindestens die wirksame Tagesmenge der erfindungsgemäßen Verbindungen, z. B. 30 - 3000, vorzugsweise 50 - 1000 mg enthalten.

Die erfindungsgemäßen Verbindungen können auch in Kombination mit anderen antiviralen Mitteln und Immunstimulantien, wie Interferonen, verabreicht werden. Im folgenden wird die Prüfung auf antivirale Eigenschaften und die Herstellung der erfindungsgemäßen Verbindungen der allgemeinen Formel I beschrieben:

In vitro-Versuche und -Ergebnisse:
HSV-1, HSV-2, HCMV, VZV

Die antivirale Aktivität der Prüfsubstanzen gegen verschiedene humanpathogene DNAund RNA-Viren wurde in Zellkulturtestsystemen untersucht. Als Standardpräparate dienen Aciclovir (HSV-1, HSV-2) und Ganciclovir (CMV, VZV).

Die Testsubstanzen werden für den Versuch in Ethanol auf eine Endkonzentration von 1 mg/ml vorverdünnt, weitere Verdünnungsschritte erfolgen in Dulbecco's Minimal Essential Medium (MEM). Für den Versuch werden 3er-Verdünnungsreihen der Testsubstanzen in 96-Napf-Mikrotiterplatten angefertigt. Affennierenzellen (Vero) in Medium 199 (5 % FCS) oder humane embryonale diploide Lungenfibroblastenzellen MRC-5 (Whittaker) werden in einer Konzentration von 2x104 Zellen/Napf hinzugefügt und 3 h bei 37°C (5 % CO₂) inkubiert.

Die Versuchsansätze enthalten die Prüfsubstanz in Konzentrationen von 400 µg/ml bis 0,18 µg/ml in MEM, ergänzt durch 4 % FCS (HeLa) bzw. 2 % FCS (Vero), 100 U/ml Penicillin G und 100 µg/ml Streptomycin.

Die Zellen werden dann mit Herpes simplex Virus Typ 1 (comeae), Herpes simplex Virus Typ 1 VR733, Herpes simplex Virus Typ 1 VR539 (hominis), Herpes simplex Virus Typ 2 VR734 (MS), humanem Cytomagalovirus VR977 oder Varizella Zoster Virus VR586 infiziert in Konzentrationen, bei denen der Zellrasen innerhalb von 3 Tagen (CMV: 7 Tage, VZV: 10 Tage) vollkommen zerstört wird (z.B. HSV-1 corneae: 0,035 PFU/Vero-Zelle, HSV-2: 0,066 PFU/Vero-Zelle).

Die Inkubation der Kulturen erfolgt bei 37°C in Gegenwart von 5 % CO₂. Alle Untersuchungen werden als Doppelbestimmungen durchgeführt, Kontrollen werden nur eimal pro Platte bestimmt.

Nach 24 h Inkubation wird die Cytotoxizität der Prüfsubstanzen durch mikroskopische Begutachtung der Zellkulturen bestimmt. Als Dosis tolerata maxima (DTM) wird die höchste Präparatkonzentration bezeichnet, die unter den genannten Versuchsbedingungen noch keine mikroskopisch erkennbaren Zellschädigungen hervorruft.

Die unbehandelten Infektionskontrollen zeigen nach weiteren 48 h Inkubation (HCMV nach 6 Tagen, VZV nach 9 Tagen) einen kompletten cytopathogenen Effekt (CPE). Nach mikroskopischer Begutachtung der Zellkulturen (HSV, HCMV, VZV) werden diese dann mit Neutralrot entsprechend dem Vitalfärbungsverfahren nach Finter (1966) angefärbt (nur HSV). Die antivirale Aktivität einer Testsubstanz wird definiert als minimale Hemmkonzentration (MHK), die benötigt wird, um 50 % der Zellen vor dem virusbedingten cytopathogenen Effekt zu schützen.

Die Ergebnisse sind in der folgenden Tabelle zusammengefaßt:

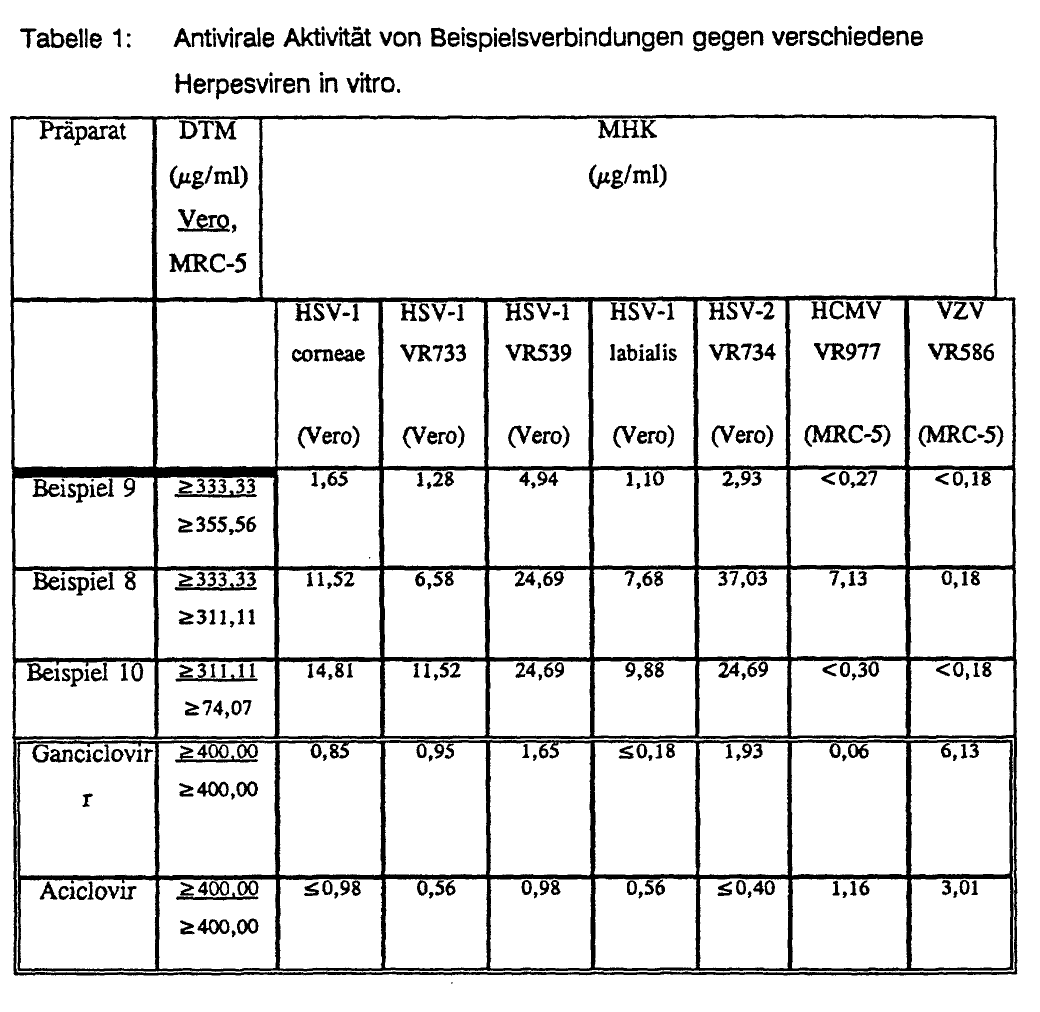

NMRI-Mäuse, spezifisch pathogenfrei, im Gewicht von 15-18 g wurden intraperitoneal mit etwa 50 LD₅₀-Dosen HSV-1 (gefrorenes Material aus der Zellkultur) infiziert. Die Mäuse wurden einmal 3 Stunden nach der Infektion und dann zweimal täglich (im Abstand von 8 Stunden) die nächsten 4 Tage mit den genannten Verbindungen in den aufgeführten Dosen intraperitoneal behandelt. Die Kontrollgruppe wurde mit physiologischer Kochsalzlösung behandelt. Die Tiere wurden zwei Wochen lang, gerechnet nach Infektionsbeginn, beobachtet. Die folgende Tabelle (Tabelle 2) zeigt die Ergebnisse dieser Untersuchung:

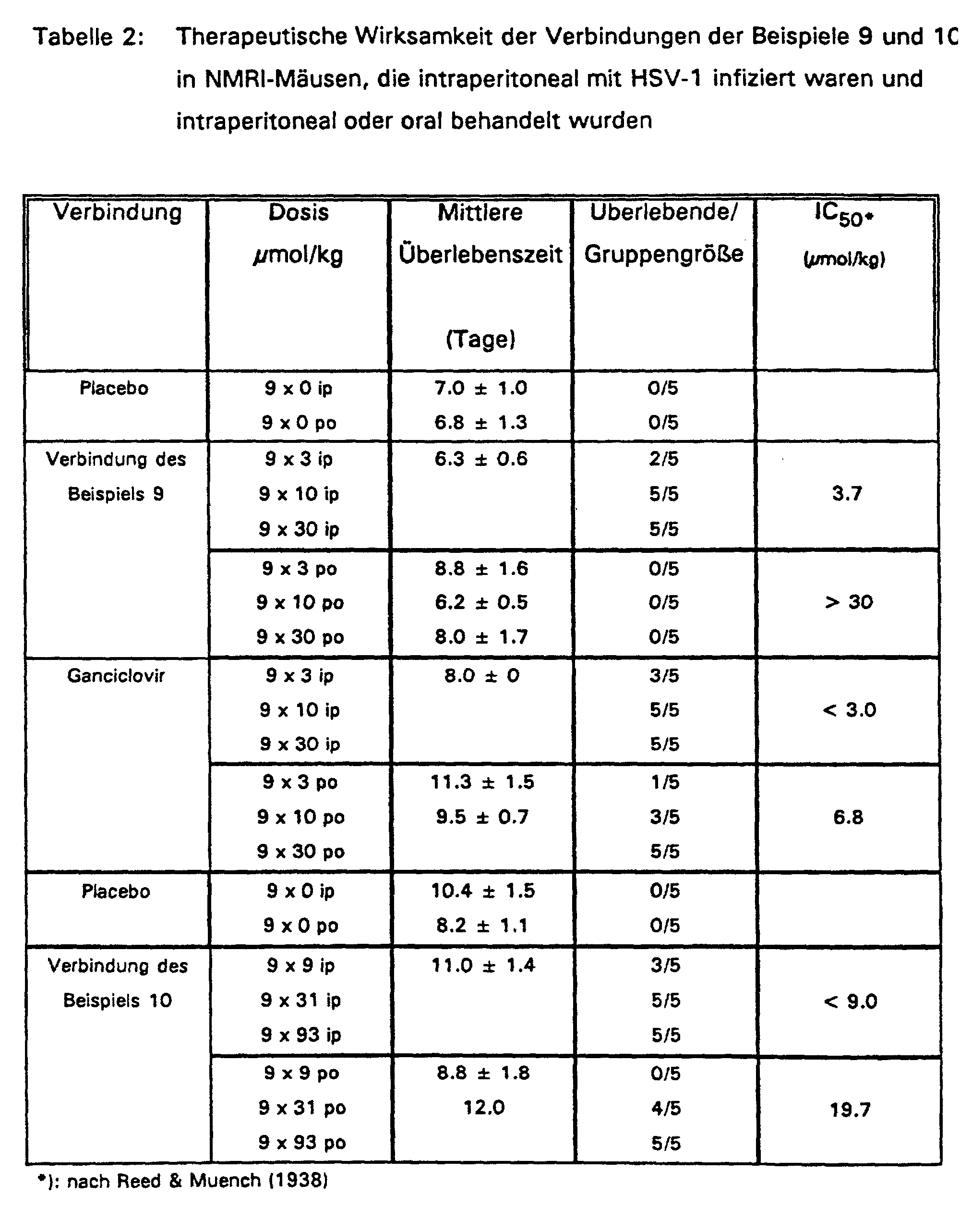

### Beispiele:

### 1. Verbindung der Formel I, worin X = Hydroxy, Y = Acetamido, R¹ = Isopropoxymethyl, R² = Isopropoxy und R³ = Wasserstoff ist:

3.88 g (0.02 mol) 5-Acetamido-7-hydroxy-1H-1,2,3-triazolo[4,5-d]pyrimidin (N²-Acetyl-8-azaguanin) [hergestellt durch Umsatz von 5-Amino-7-hydroxy-1H-1,2,3-triazolo[4,5-d]pyrimidin (8-Azaguanin) mit Acetanhydrid und katalytischen Mengen N,N-Dimethylaminopyridin in der Siedehitze und nachfolgender Monodeacetylierung mit gesättigter wäßriger Natriumhydrogencarbonatlösung, 79.5% d. Th., Fp. > 280°C, ¹H NMR (200 MHz, d₆-DMSO) d[ppm]: 16.0 (s, breit, 1H), 12.15 (s, 1H), 11.79 (s, 1H), 2.20 (s, 3H)] werden mit 20 ml trockenem Xylol, 20 ml Hexamethyldisilazan und 0.2 g Ammoniumsulfat 1 h unter einer Argonatmosphäre unter Rühren zum Rückfluß erhitzt. Danach wird das Lösungsmittel und überschüssiges Hexamethyldisilazan im Vakuum abdestilliert, der Rückstand in 20 ml trockenem 1,2-Dichlorethan gelöst und zu einer Lösung von 7.3 g (0.02 mol) Formaldehyd-bis-(1,3-bis-isopropoxy-2-propyl)-acetal [hergestellt wie in DE 4020481 A1 beschrieben] in 20 ml trockenem 1,2-Dichlorethan bei -30°C zugefügt. Sodann werden zu dieser Lösung bei -30°C und unter einer Argonatmosphäre langsam 4.7 ml (0.025 mol) Trifluormethansulfonsäuretrimethylsilylester zugetropft. Die resultierende Lösung wird 5 h bei -30 bis -20°C gerührt und anschließend in eine kalte Natriumchlorid-gesättigte konzentrierte Natriumhydrogencarbonatlösung eingerührt. Die entstandene Suspension wird filtriert, der Rückstand mit Dichlormethan gewaschen und die organische Phase abgetrennt. Die wäßrige Phase wird mehrmals mit Dichlormethan extrahiert; die organischen Phasen werden vereinigt, über Natriumsulfat getrocknet und im Vakuum eingeengt. Der ölige Rückstand wird chromatographiert (Kieselgel, Laufmittel: Essigsäureethylester/n-Heptan 2/1), und man erhält als erste Fraktion 3.18 g (41.6% d. Th.) der Titelverbindung des Beispiels 1, 5-Acetamido-7-hydroxy- 1H-1-{[(1,3-bis-isopropoxy)-2-propoxy]methyl}-1,2,3-triazolo[4,5-d]pyrimidin, als farblose Kristalle mit dem Schmelzpunkt 156°C. ¹H NMR (200 MHz, d₆-DMSO) d[ppm]: 12.42 (s, 1H), 11.80 (s, 1H), 6.02 (s, 2H), 4.0 - 3.85 (m, 1H), 3.5 - 3.2 (m, 6H), 2.2 (s, 3H), 0.94 (m, 12H). Als zweite Fraktion werden 0.93 g (12.2% d. Th.) der isomeren Verbindung 5- Acetamido-7-hydroxy-3H-3-{[(1,3-bis-isopropoxy)-2-propoxy]-methyl}-1,2,3-triazolo[4,5-d]pyrimidin, als farblose Kristalle mit dem Schmelzpunkt 95°C und dem ¹H NMR-Spektrum (200 MHz, d₆-DMSO) d[ppm]: 12.21 (s, breit, 1H), 12.05 (s, breit, 1H), 5.85 (s, 2H), 3.9 - 3.79 (m, 1H), 3.5 - 3.2 (m, 6H), 2.12 (s, 3H), 0.95 (m, 12H) erhalten.

### 4. Verbindung der Formel I, worin X = Hydroxy, Y = Amino, R¹ = Hydroxymethyl, R² = Hydroxy und R³ = Wasserstoff ist:

0.25 g (0.84 mmol) der Verbindung des Beispiels 3 werden in 10 ml 40%iger wäßriger Methylaminlösung 2 h zum Rückfluß erhitzt und danach im Vakuum vollständig eingeengt. Der Rückstand wird in wenig warmen Wassers gelöst, mit 2N Essisäure neutralisiert und im Vakuum vollständig eingeengt. Der Rückstand wird in kaltem Ethanol suspendiert, abgesaugt und mit Ethanol und Diethylether gewaschen. Nach Kristallisation aus Wasser erhält man 0.2 g (93% d.Th.) 5-Amino-7-hydroxy-1H- 1-[(1,3-dihydroxy-2-propoxy)methyl]-1,2,3-triazolo[4,5-d]pyrimidin als farblose Kristalle mit dem Schmelzpunkt >290°C. ¹H NMR (200 MHz, d₆-DMSO) d[ppm]: 11.25 (s, 1H), 6,50 (s, 2H), 5.95 (s, 2H), 4.55 (t, 2H), 3.70 (m, 1H), 3.45 - 3.10 (m, 4H).

### 5. Verbindung der Formel I, worin X = Mercapto, Y = Acetamido, R¹ = Isopropoxymethyl, R² = Isopropoxy und R³ = Wasserstoff ist:

5.73 g (15 mmol) der Verbindung des Beispiels 1 werden in 225 ml trockenem Toluol mit 6.7 g (16.5 mmol) 2,4-Bis-(4-methoxyphenyl)-1,3-dithia-2,4-diphosphetan- 2,4-disulfid unter einer Argonatmosphäre 3 h bei 85°C gerührt. Anschließend wird die Reaktionsmischung im Vakuum eingeengt und chromatographiert (Kieselgel; Laufmittel: Essigsäureethylester/n-Heptan 2/1). Man erhält 2.7 g (45% d. Th.) 5-Acetamido-7-mercapto-1H-1-{[(1,3-bis-isopropoxy)-2-propoxy]methyl}-1,2,3-triazolo[4,5-d]pyrimidin, als gelbliche Kristalle mit dem Schmelzpunkt 91°C. ¹H NMR (200 MHz, d₆-DMSO) d[ppm]: 13.79 (s, 1H), 12.05 (s, 1H), 6.35 (s, 2H), 4.05 - 3.92 (m, 1H), 3.5 - 3.24 (m, 6H), 2.24 (s, 3H), 0.94 (m, 12H).

### 6. Verbindung der Formel I, worin X = Wasserstoff, Y = Acetamido, R¹ = Isopropoxymethyl, R² = Isopropoxy und R³ = Wasserstoff ist:

0.6 g (1.5 mmol) der Verbindung des Beispiels 5 werden in 60 ml absoluten Ethanols gelöst, mit ca. 3 g Raney-Nickel (mit Ethanol gewaschen) versetzt, 3h zum Rückfluß erhitzt, noch warm vom Raney-Nickel abfiltriert und im Vakuum eingeengt. Nach Chromatographie an Kieselgel mit Essigsäureethylester/n-Heptan 2/1 werden 0.25 g (45.5% d. Th.) 5-Acetamido-1H-1-{[(1,3-bis-isopropoxy)-2-propoxy]methyl}- 1,2,3-triazolo[4,5-d]pyrimidin als farblose Kristalle mit dem Schmelzpunkt 104°C erhalten. ¹H NMR (200 MHz, d₆-DMSO) d[ppm]: 10.88 (s, 1H), 9.55 (s, 1H), 6.25 (s, 2H), 3.82 - 3.72 (m, 1H), 3.45 - 3.10 (m, 6H), 2.22 (s, 3H), 0.88 (d, 12H).

### 7. Verbindung der Formel I, worin X = Wasserstoff, Y = Amino, R¹ = Isopropoxymethyl, R² = Isopropoxy und R³ = Wasserstoff ist:

0.5 g (1.37 mmol) der Verbindung des Beispiels 6 werden wie in Beispiel 2 mit 40%iger wäßriger Methylaminlösung behandelt und liefern nach chromatographischer Reinigung (Kieselgel; Laufmittel:
Essigsäureethylester/Methanol 9/1) 0.40 g (90.1% d. Th.) 5-Amino-1H-1-{[(1,3-bis-isopropoxy)-2-propoxy]methyl}- 1,2,3-triazolo[4,5-d]pyrimidin als farblose Kristalle mit dem Schmelzpunkt 87°C. ¹H NMR (270 MHz, d₆-DMSO) d[ppm]: 9.18 (s, 1H), 6.92 (s, 2H), 6.10 (s, 2H), 3.77 - 3.67 (m, 1H), 3.47 - 3.25 (m, 6H), 0.92 (d, 12H).

### 8. Verbindung der Formel I, worin X = Wasserstoff, Y = Acetamido, R¹ = Hydroxymethyl, R² = Hydroxy und R³ = Wasserstoff ist:

1.15 g (3.14 mmol) der Verbindung des Beispiels 6 werden in 20 ml trockenem Dichlormethan (Argonatmosphäre) gelöst. Zu dieser Lösung werden bei -60°C unter Rühren langsam 12.56 ml (12.56 mmol) einer 1-molaren Lösung von Bortrichlorid in Dichlormethan zugetropft. Die resultierende Lösung wird 5 h bei -40 bis -30°C gerührt; danach tropft man bei -60°C langsam eine Mischung von 12.5 ml Methanol mit 12.5 ml Dichlormethan zu, bevor man ca. 5 ml Triethylamin zusetzt. Die Lösung wird 1 h bei Raumtemperatur gerührt und danach im Vakuum zur Trockne eingeengt. Der Rückstand wird chromatographisch gereinigt (Kieselgel; Laufmittel: Essigsäureethylester/Isopropanol/Wasser 4/3/0.3). Die Chromatographie liefert 0.55 g (62.1% d. Th.)
5-Acetamido-1H-1-[(1,3-dihydroxy-2-propoxy)methyl]-1,2,3-triazolo[4,5-d]-pyrimidin als farblose Kristalle mit dem Schmelzpunkt 167-168°C, ¹H NMR (200 MHz, d₆-DMSO) d[ppm]: 10.9 (s, 1H), 9.58 (s, 1H), 6.27 (s, 2H), 4.63 (t, 2H), 3.65 - 3.5 (m, 1H), 3.5 - 3.18 (m, 4H), 2.23 (s, 3H).

### 9. Verbindung der Formel I, worin X = Wasserstoff, Y = Amino, R¹ = Hydroxymethyl, R² = Hydroxy und R³ = Wasserstoff ist:

0.5 g (1.78 mmol) der Verbindung des Beispiels 8 werden in einer Mischung aus 10 ml Methanol mit 10 ml 40%iger wäßriger Methylaminlösung unter Rühren gelöst und 2 h zum Rückfluß erhotzt. Die resultierende Lösung wird mit etwas wäßrigem Methanol verdünnt, mit Aktivkohle aufgekocht, filtriert und der Rückstand zweimal mit Ethanol gewaschen. Die vereinigten Filtrate werden eingeengt, der Rückstand wird mit kaltem Ethanol angerührt, und die erhaltene Suspension wird filtriert und mit etwas Ethanol und Diethylether gewaschen. Der Rückstand wird aus Ethanol umkristallisiert und liefert 0.35 g (81.9% d. Th.) 5-Amino-1H-1-[(1,3-dihydroxy-2-propoxy)methyl]-1,2,3-triazolo[4,5-d]pyrimidin als farblose Kristalle mit dem Schmelzpunkt 161°C. ¹H NMR (200 MHz,
d₆-DMSO) d[ppm]: 9.19 (s, 1H), 6.91 (s, 2H), 6.13 (s, 2H), 4.61 (t, 2H), 3.60 - 3.23 (m, 5H).

### 10. Verbindung der Formel I, worin X = Wasserstoff, Y = Amino, R1 = Acetoxymethyl, R2 = Acetoxy und R3 = Wasserstoff ist:

0.24 g (1 mmol) der Verbindung des Beispiels 9 werden in 10 ml wasserfreiem Acetonitril suspendiert, und unter Rühren werden 40 mg 4-Dimethylaminopyridin und 0.6 g (2.9 mmol) Dicyclohexylcarbodiimid und 0.17 ml (0.175 g, 2.9 mmol) Eisessig zugegeben. Die Mischung wird 2 h bei Raumtemperatur gerührt, sodann mit 23.5 ml Isopropanol und 23.5 ml Wasser versetzt und weitere 15 min gerührt. Der Niederschlag (Dicyclohexylharnstoff) wird abfiltriert, der Rückstand mit Tetrahydrofuran gewaschen und das klare Filtrat wird vollständig eingeengt. Der kristalline Rückstand wird über Kieselgel mit einem Gemisch aus 20 Teilen Essigsäureethylester mit 1 Teil Methanol chromatographiert. Auf diese Weise erhält man - nach Kristallisation aus Isopropanol - 0.29 g (89.5% d. Th.) 5-Amino-1H-1-[(1,3-diacetoxy-2-propoxy)methyl]-1,2,3-triazolo[4,5-d]pyrimidin als farblose Kristalle mit dem Schmelzpunkt 143 - 144°C. ¹H NMR (200 MHz, d₆-DMSO) δ[ppm]: 9.2 (s, 1H), 6.97 (s, 2H), 6.12 (s, 2H), 4.15 - 3.95 (m, 5H), 1.79 (s, 3H).

## Patentansprüche

1. Verbindung der Formel I 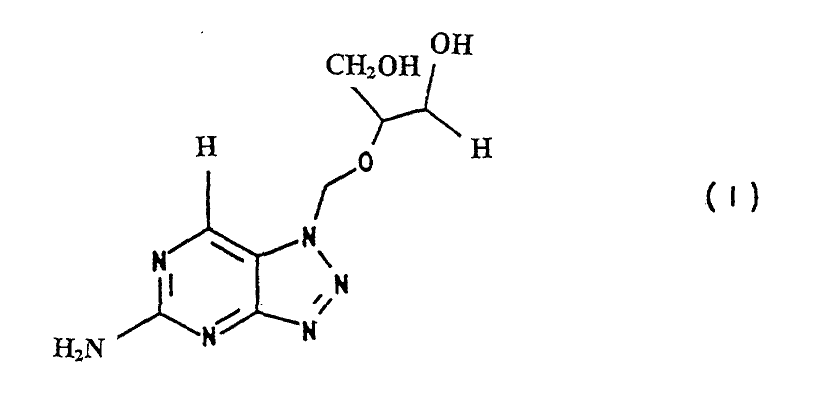 deren tautomere Formen sowie deren physiologisch verträgliche Salze.

2. Verfahren zur Herstellung von Verbindungen der Formel I gemäß Anspruch 1, **dadurch gekennzeichnet, daß** man eine Verbindung der Formel II, 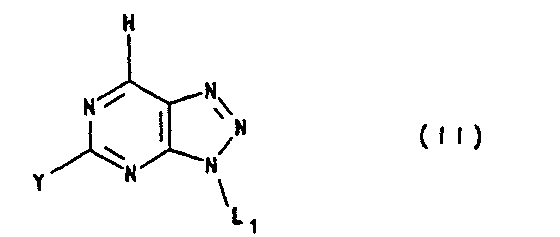 worin
Y = Trialkylsilylamino,
und die austretende Gruppe L₁ = Acyloxy oder Trialkylsilyl bedeuten
mit einer Verbindung der Formel III, 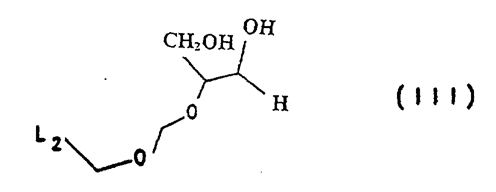 worin
die austretende Gruppe L₂ = Halogen, oder die C₁-C₆-Alkylthio- oder C₁-C₆-Alkylsulfinyl- oder C₁-C₆-Alkylsulfonyl-Gruppierung, oder die Methylsulfinyl- oder die Methylsulfonyl-Gruppierung, oder C₁-C₈-Acyloxy oder Benzoyloxy, oder aber -O-CHR¹-CHR²R³ bedeuten (wobei in diesem Fall die Verbindung der Formel III ein symmetrische Formaldehydacetal darstellt), in einem aprotischen Lösungsmittel wie Benzol, Toluol, Xylol, Acetonitril, Dichlormethan oder 1,2-Dichlorethan oder Gemischen davon, unter einer Schutzgasatmosphäre aus Argon oder Stickstoff, in Gegenwart einer Säure, oder in Gegenwart von Jod oder Carbonsäure- oder Alkylsulfonsäuretrialkylsilylestern, wobei die Mengen dieser Reagenzien 0.01 bis 10, Äquivalente, bezogen auf die Menge der jeweils eingesetzten Verbindung der Formel II, betragen, bei Temperaturen zwischen -70°C und +40°C, während 1 bis 24 Stunden, umsetzt.

3. Verfahren gemäß Anspruch 2, worin L1=Trimethylsilyl; L2=Chlor, Methylthio oder Acetoxy bedeutet, in dem man in Gegenwart einer Lewis-Säure wie Aluminiumtrichlorid, Aluminiumsulfat, Bortrifluorid, Eisentrichlorid, Galliumtrichlorid, Zinntetrachlorid oder Titantetrachlorid, oder in Gegenwart von Trifluormethansulfonsäuretrimethylsilylester, bei -40° C bis +20° C, während 2-8 Stunden umsetzt, wobei die Mengen dieser Reagenzien 0,3 bis 1,3 Äquivalente betragen.

4. Verwendung von Verbindungen gemäß Anspruche-1 als Arzneimittel.

5. Arzneimittel mit einem Gehalt an mindestens einer Verbindung der Formel I gemäß Anspruch 1 .

6. Verwendung von Verbindungen gemäß Anspruch 1 zur Herstellung von Arzneimitteln zur Behandlung von Viruserkrankungen.

7. Verfahren zur Herstellung von Arzneimitteln gemäß Anspruch 5, **dadurch gekennzeichnet, daß** man mindestens eine Verbindung der Formel I gemäß Anspruch 1 gegebenenfalls mit geeigneten Hilfs- und/oder Trägerstoffen in eine geeignete Darreichungsform bringt.

## Claims

1. Compound of the formula I 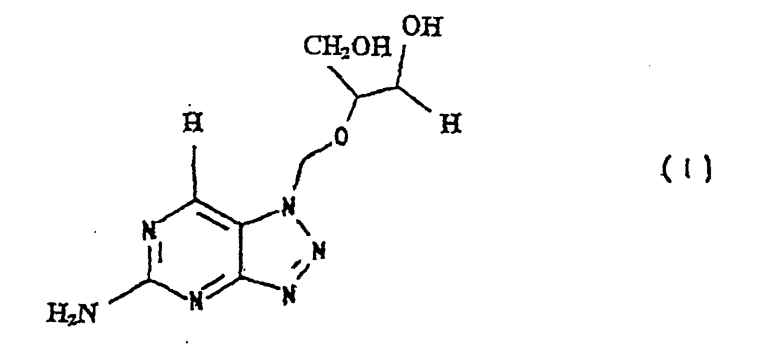 tautomeric forms thereof, and physiologically tolerated salts thereof.

2. Process for the preparation of compounds of the formula I according 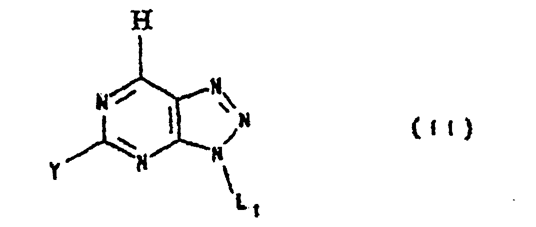 to Claim 1, **characterized in that** a compound of the formula II in which
Y = trialkylsilylamino,
and the leaving group L₁ = acyloxy or trialkylsilyl,
is reacted with a compound of the formula III 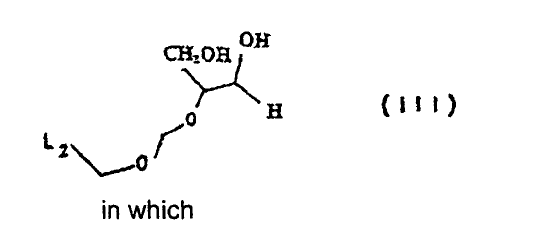 the leaving group L₂ = halogen, or the C₁-C₆-alkylthio or C₁-C₆-alkylsulphinyl or C₁-C₆-alkylsulphonyl group, or the methylsulphinyl or methylsulphonyl group, or C₁-C₈-acyloxy or benzoyloxy, or alternatively -O-CHR¹-CHR²R³ (where in this case the compound of the formula III is a symmetrical formaldehyde acetal), in an aprotic solvent, such as benzene, toluene, xylene, acetonitrile, dichloromethane or 1,2-dichloroethane, or mixtures thereof, under a protective-gas atmosphere comprising argon or nitrogen, in the presence of an acid, or in the presence of iodine or trialkylsilyl carboxylates or alkylsulphonates, where the amounts of these reagents are from 0.01 to 10 equivalents, based on the amount of compound of the formula II employed in each case, at temperatures between -70°C and +40°C, for from 1 to 24 hours.

3. Process according to Claim 2, in which L₁ = trimethylsilyl; L₂ = chlorine, methylthio or acetoxy, where the reaction is carried out in the presence of a Lewis acid, such as aluminium trichloride, aluminium sulphate, boron trifluoride, iron trichloride, gallium trichloride, tin tetrachloride or titanium tetrachloride, or in the presence of trimethylsilyl trifluoromethanesulphonate, at from -40°C to +20°C, for 2-8 hours, where the amounts of these reagents are from 0.3 to 1.3 equivalents.

4. Use of compounds according to Claim 1 as medicaments.

5. Medicaments having a content of at least one compound of the formula I according to Claim 1.

6. Use of compounds according to Claim 1 for the preparation of medicaments for the treatment of viral diseases.

7. Process for the preparation of medicaments according to Claim 5, **characterized in that** at least one compound of the formula I according to Claim 1 is converted into a suitable administration form, if desired with suitable adjuvants and/or excipients.

## Revendications

1. Composé de formule I 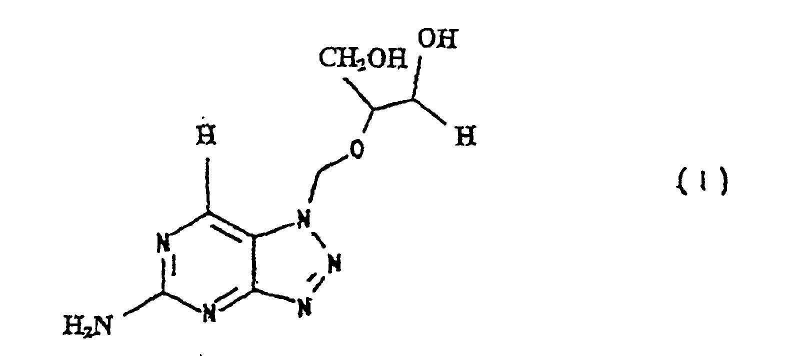 les formes tautomères de celui-ci, et les sels physiologiquement tolérés de celui-ci.

2. Procédé de préparation de composés de formule I selon la revendication 1, **caractérisé en ce qu'**un composé de formule II 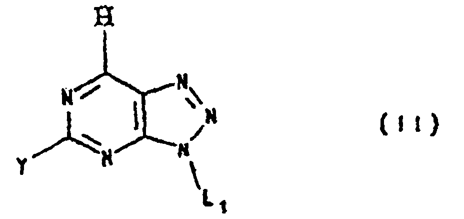 dans laquelle
Y = trialkylsilylamino,
et le groupement partant L₁ = acyloxy ou trialkylsilyle,
entre en réaction avec un composé de formule III 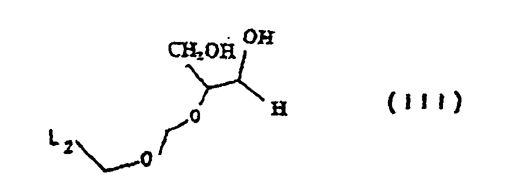 dans laquelle
le groupement partant L₂ = halogène, ou le groupement alkyl-C₁-C₆-thio ou alkyl-C₁-C₆-sulfinyle ou alkyl-C₁-C₆-sulfonyle, ou le groupement méthylsulfinyle ou méthylsulfonyle, ou acyl-C₁-C₈-oxy ou benzoyloxy, ou de manière alternative -O-CHR¹-CHR²R³ (où dans ce cas le composé de formule III représente un acétal de formaldéhyde symétrique), dans un solvant aprotique, tel que le benzène, le toluène, le xylène, l'acétonitrile, le dichlorométhane ou le 1,2-dichloroéthane, ou des mélanges de ceux-ci, sous une atmosphère gazeuse protectrice comprenant de l'argon ou de l'azote, en présence d'un acide, ou en présence d'iode ou de carboxylates ou d'alkylsulfonates de trialkylsilyle, où les quantités de ces réactifs vont de 0,01 à 10 équivalents, rapportées à la quantité de composé de formule II employé dans chaque cas, à des températures comprises entre -70°C et +40°C, pour une durée allant de 1 à 24 heures.

3. Procédé selon la revendication 2, dans lequel L₁ = triméthylsilyle ; L₂ = chlore, méthylthio ou acétoxy, où la réaction est réalisée en présence d'un acide de Lewis, tel que le trichlorure d'aluminium, le sulfate d'aluminium, le trifluorure de bore, le trichlorure de fer, le trichlorure de gallium, le tétrachlorure d'étain ou le tétrachlorure de titane, ou en présence de trifluorométhanesulfonate de triméthylsilyle, à une température allant de -40°C à +20°C, pendant 2-8 heures, où les quantités de ces réactifs vont de 0,3 à 1,3 équivalents.

4. Utilisation des composés selon la revendication 1 comme médicaments.

5. Médicaments ayant une teneur en au moins un composé de formule I selon la revendication 1.

6. Utilisation des composés selon la revendication 1 pour la préparation de médicaments pour le traitement de maladies virales.

7. Procédé de préparation de médicaments selon la revendication 5, **caractérisé en ce qu'**au moins un composé de formule I selon la revendication 1 est mis sous une forme d'administration appropriée, le cas échéant avec des adjuvants et/ou des excipients appropriés.
